(19) Europäisches Patentamt
European Patent Office
Office européen des brevets

(11) Veröffentlichungsnummer: **0 070 384**
**B1**

# (12) EUROPÄISCHE PATENTSCHRIFT

(45) Veröffentlichungstag der Patentschrift: **05.12.84**

(51) Int. Cl.³: **C 07 D 249/18**, A 01 N 43/64

(21) Anmeldenummer: **82104913.7**

(22) Anmeldetag: **04.06.82**

## (54) Benzotriazole, ihre Herstellung und ihre Verwendung als Fungizide.

(30) Priorität: **26.06.81 DE 3125112**

(43) Veröffentlichungstag der Anmeldung: **26.01.83 Patentblatt 83/4**

(45) Bekanntmachung des Hinweises auf die Patenterteilung: **05.12.84 Patentblatt 84/49**

(84) Benannte Vertragsstaaten: **AT BE CH DE FR GB IT LI LU NL**

(56) Entgegenhaltungen: **EP - A - 0 029 160**

(73) Patentinhaber: **CELAMERCK GmbH & Co. KG, Binger Strasse 173, D-6507 Ingelheim am Rhein (DE)**

(72) Erfinder: **Sehring, Richard, Dr. Dipl.-Chem., Frankenstrasse 13, D-6507 Ingelheim (DE)**
Erfinder: **Buck, Wolfgang, Dr. Dipl.-Chem., In der Dörrwiese 37, D-6507 Ingelheim (DE)**
Erfinder: **Raddatz, Erich, Dr. Dipl.-Landwirt, Carrera 1-Oe No. 5-265, Cali (CO)**
Erfinder: **Lust, Sigmund, Dr., Klappacher Strasse 2 f, D-6100 Darmstadt (DE)**

**EP 0 070 384 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft Benzotriazole der allgemeinen Formel I:

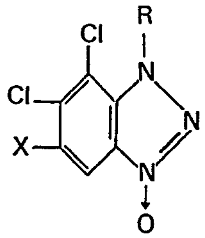

(I)

in der

R für einen geradkettigen oder verzweigten Alkylrest mit 1 bis 4 C-Atomen oder einen Allylrest,

X für ein Wasserstoff- oder Chloratom steht, die Herstellung dieser Verbindungen und die Verwendung dieser Verbindungen bei der Bekämpfung von phytopathogenen Pilzen.

Die Verbindungen können überraschenderweise erhalten werden, indem man

a) ein bekanntes Benzotriazol der allgemeinen Formel IIa:

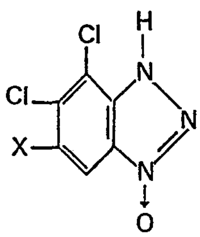

(IIa)

bzw. die tautomere Verbindung der allgemeinen Formel IIb:

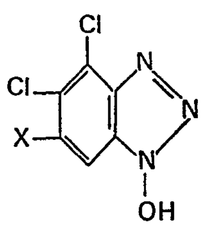

(IIb)

worin X die obige Bedeutung hat, mit einem der Einführung des Restes R entsprechenden Schwefel- bzw. Sulfonsäureester, zweckmässig in einem inerten Lösungsmittel erhitzt, oder dass man

b) eine Verbindung der allgemeinen Formel III:

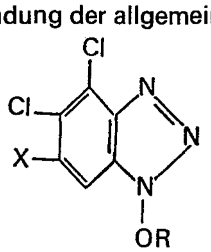

(III)

worin R und X die obige Bedeutung haben, in Gegenwart einer geringen Menge eines Schwefel- oder Sulfonsäureesters oder einer geringen Menge eines üblichen Phasentransferkatalysators erhitzt.

Bei Verfahren a benutzt man ggf. als Reaktionsmedium ein inertes organisches Lösungsmittel, wie Toluol, Benzol, Xylol, Chlorbenzol, und erhitzt bis zu mehreren Stunden lang auf Temperaturen zwischen etwa 80 und 150°C.

Bei Verfahren b kann auf ein Lösungsmittel verzichtet werden, da die Reaktion sich auch gut in der Schmelze durchführen lässt. Auch hier wird auf Temperaturen zwischen etwa 80 und 150°C erhitzt. Im allgemeinen verläuft die Reaktion in kurzer Zeit. Als Katalysator eignen sich besonders Schwefel- bzw. Sulfonsäureester, wie sie auch zur Einführung des Restes R bei Verfahren a benutzt werden. Einfacher ist es im allgemeinen, Dimethylsulfat oder p-Toluolsulfonsäuremethylester als Katalysator zu verwenden.

Als Katalysatoren für Verfahren b geeignete Phasentransferkatalysatoren sind beispielsweise der Monographie von E.V. und S.S. Dehmlow, „Phase Transfer Catalysis", Verlag Chemie, Weinheim (1980), insbesondere S. 38 sowie S. 39-43, zu entnehmen.

Zu nennen sind beispielsweise Triäthylbenzylammoniumchlorid oder -bromid; Tetrabutylammoniumchlorid, -bromid, -hydrogensulfat, -hydroxid; Methyltrioctylammoniumchlorid; Tetrabutylphosphoniumbromid.

Die Katalysatormenge kann in weiten Grenzen variiert werden und zwischen ca. 1:10 und 1:1000 liegen (Gewichtsverhältnis Katalysator/Ausgangsstoff). Insbesondere bei grösseren Ansätzen kann die untere Grenze noch erheblich gesenkt werden.

Die Verbindung der allgemeinen Formel I zeichnen sich durch eine starke fungizide Wirkung gegen phytopathogene Pilze aus. Hervorzuheben ist die Wirkung gegen wichtige Krankheitserreger an Reis, etwa *Pellicularia, Helminthosporium* und besonders *Pyricularia oryzae*. Gegen diese Pilze sind weder die Ausgangsstoffe der allgemeinen Formeln IIa/IIb noch die der allgemeinen Formel III wirksam.

Für die Anwendung werden die erfindungsgemässen Wirkstoffe zu üblichen Formulierungen verarbeitet, z.B. zu Suspensionspulvern, Stäuben, Granulaten, Emulsions- bzw. Lösungskonzentraten.

Aus den Konzentraten werden Spritzbrühen mit im allgemeinen zwischen 0,01 und 0,1 Gew.-% Wirkstoff durch Vermischen mit Wasser hergestellt.

Beispiele für die Formulierung:

1. *Emulsionskonzentrat*

5,0 Gew.-Teile Wirkstoff gemäss der Erfindung
3,4 Gew.-Teile epoxidiertes Pflanzenöl
13,4 Gew.-Teile eines Kombinationsemulgators aus Fettalkoholpolyglykoläther und Calciumalkylarylsulfonat
40,0 Gew.-Teile Dimethylformamid
38,2 Gew.-Teile Xylol

Die Komponenten werden vermischt und für die Anwendung mit Wasser auf eine Wirkstoffkonzentration von 0,01 bis 0,1 Gew.-% verdünnt.

2. *Suspensionspulver*

10 Gew.-Teile Wirkstoff gemäss der Erfindung
3 Gew.-Teile Natriumfettalkoholsulfonat
5 Gew.-Teile Salze von Naphthalinsulfonsäure/

Formaldehyd-Kondensat
82 Gew.-Teile Kaolin

Die nach Beispiel 1 erhaltene Verbindung wurde unter tropischen Bedingungen an Saatreis untersucht. Saatreisreihen zwischen älteren, infizierten Reihen wurden am 18., 23. und 29. Tag nach der Saat mit einer Spritzbrühe behandelt, die 500 ppm Wirkstoff enthielt. Als Vergleich diente die Wirkung eines üblichen *Pyricularia*-Mittels sowie die nur mit Wasser behandelte Kontrolle. Das Ergebnis ist in der Tabelle festgehalten:

| Wirkstoff | Konzentration (ppm) | Prozentualer Infektionsgrad<br>0 | <br>6 | <br>10 | <br>14 |
|---|---|---|---|---|---|
| | | Tage nach letzter Behandlung | | | |
| Beispiel 1 | 500 | 5 | 4 | 6 | 14 |
| Vergleichsmittel | 1000 | 4 | 8 | 11 | 18 |
| Kontrolle (Wasser) | | 5 | 31 | 40 | 42 |

Die erfindungsgemässe Verbindung erweist sich mit der halben Aufwandmenge als stärker wirksam als die Vergleichsverbindung und erweist sich als gut verträglich.

*Beispiel 1:*

*3-Methyl-4,5-dichlorbenzotriazol-1-N-oxid*

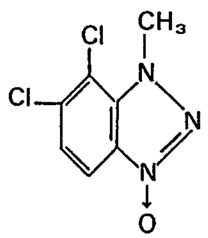

153 g (0,75 mol) 4,5-Dichlor-1-hydroxibenzotriazol werden in 500 ml Toluol suspendiert und mit 99 g Dimethylsulfat versetzt. Man lässt ca. 1 h unter Rückfluss kochen, wobei sich zwei Schichten bilden. Das abgekühlte Reaktionsgemisch wird mit Eiswasser versetzt, die Toluolschicht dann abgetrennt. Aus der wässerigen Phase wird das ausgefallene Produkt abgesaugt, mit Dimethylformamid heiss ausgerührt, abgekühlt und abgesaugt. Man wäscht mit kaltem Äthanol nach. Ausbeute 105 g (68% d. Th.). Ein weiterer Anteil des Reaktionsprodukts kann aus der Toluolphase isoliert werden. Fp. 164°C.

*Analyse*

| | | | |
|---|---|---|---|
| Berechnet: | C 38,50 | H 2,29 | N 19,25% |
| Gefunden: | C 38,69 | H 2,18 | N 19,02% |

*Beispiel 2:*

*3-Methyl-4,5-dichlorbenzotriazol-1-N-oxid*

5,4 g (0,025 mol) 4,5-Dichlor-1-methoxibenzotriazol und 0,12 g (0,001 mol) Dimethylsulfat werden 1 h auf 140°C erhitzt. Beim Aufheizen entsteht zunächst bei 100°C eine Schmelze, die sich dann bei 110°C wieder verfestigt.

Das Reaktionsprodukt wird nach dem Erkalten aus 15 ml Äthanol umkristallisiert; Fp. 164°C, Ausbeute 92,5% d. Th.

Zum gleichen Resultat kommt man, wenn man anstelle des Dimethylsulfats 0,5 g Tetrabutylammoniumhydrogensulfat als Umlagerungskatalysator verwendet.

Der Ausgangsstoff kann analog „J. prakt. Chem.", *111*, S. 293 (1925) durch O-Methylierung von 1-Hydroxi-4,5-dichlorbenzotriazol mit Methyljodid in Gegenwart von Alkali erhalten werden. Entsprechend gewinnt man die anderen, als Ausgangsstoffe benötigten O-Alkyl- bzw. O-Allylverbindungen.

*Beispiel 3:*

*3-Allyl-4,5-dichlorbenzotriazol-1-N-oxid*

7,0 g 4,5-Dichlor-1-allyloxibenzotriazol und 0,10 g Dimethylsulfat werden 2 h auf 130°C erhitzt. Das Reaktionsprodukt wird aus Toluol umkristallisiert; Fp. 102°C, Ausbeute 85,3% d. Th.

Zum gleichen Ergebnis gelangt man, wenn das Dimethylsulfat a durch die gleiche Menge p-Toluolsulfonsäuremethylester oder b 0,05 g Methansulfonsäureallylester oder c 0,2 g Triäthylbenzylammoniumchlorid ersetzt wird.

Entsprechend werden erhalten:

3-Methyl-4,5,6-trichlorbenzotriazol-1-N-oxid, Fp. 224°C;

3-Äthyl-4,5-dichlorbenzotriazol-1-N-oxid, Fp. 132°C (Alkylierungsmittel p-Toluolsulfonsäureäthylester oder Umlagerung mit p-Toluolsulfonsäureäthylester);

3-n-Butyl-4,5-dichlorbenzotriazol-1-N-oxid, Fp. 62°C (Alkylierungsmittel p-Toluolsulfonsäure-n-butylester oder Umlagerung mit Methylsulfonsäureäthylester);

3-Isopropyl-4,5,6-trichlorbenzotriazol-1-N-oxid, Fp. 90°C (Alkylierungsmittel p-Toluolsulfonsäureisopropylester);

3-Äthyl-4,5,6-trichlorbenzotriazol-1-N-oxid, Fp. 154°C (Alkylierungsmittel Diäthylsulfat);

3-t-Butyl-4,5,6-trichlorbenzotriazol-1-N-oxid (Umlagerung nach Verfahren b mit Dimethylsulfat bei Verhältnis 1:100).

**Patentansprüche**

1. Verbindungen der allgemeinen Formel (I):

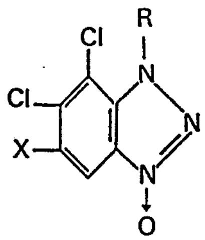

(I)

in der

R für einen geradkettigen oder verzweigten Alkylrest mit 1 bis 4 C-Atomen oder für Allyl,

X für ein Wasserstoff- oder Chloratom steht.

2. Verbindung der allgemeinen Formel (I), worin X ein Wasserstoffatom bedeutet.

3. 3-Methyl-4,5-dichlorbenzotriazol-1-N-oxid.

4. 3-Äthyl-4,5-dichlorbenzotriazol-1-N-oxid.

5. 3-n-Butyl-4,5-dichlorbenzotriazol-1-N-oxid.

6. 3-Methyl-4,5,6-trichlorbenzotriazol-1-N-oxid.

7. 3-Äthyl-4,5,6-trichlorbenzotriazol-1-N-oxid.

8. 3-Isopropyl-4,5,6-trichlorbenzotriazol-1-N-oxid.

9. 3-Allyl-4,5-dichlorbenzotriazol-1-N-oxid.

10. Verfahren zur Herstellung von Verbindungen nach den Ansprüchen 1 bis 9, dadurch gekennzeichnet, dass man

a) eine Verbindung der allgemeinen Formel (IIa) bzw. (IIb):

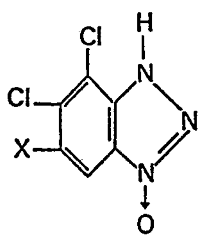

bzw.

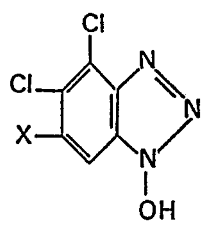

(IIa) (IIb)

worin X die obige Bedeutung hat, mit einem der Einführung des Restes R entsprechenden Schwefel- bzw. Sulfonsäureester, zweckmässig in einem inerten Lösungsmittel, mehrere Stunden erhitzt und das Reaktionsprodukt isoliert, oder dass man

b) eine Verbindung der allgemeinen Formel (III):

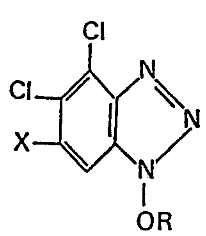

(III)

worin R und X die obige Bedeutung haben, in Gegenwart einer katalytischen Menge eines Schwefel- bzw. Sulfonsäureesters oder eines üblichen Phasentransferkatalysators erhitzt.

11. Verfahren nach Anspruch 10 b, dadurch gekennzeichnet, dass der als Katalysator benutzte Schwefel- oder Sulfonsäureester sich von einem Alkohol der Formel ROH ableitet, worin R die selbe Bedeutung hat wie in dem eingesetzten Ausgangsstoff der allgemeinen Formel (III).

12. Verfahren nach Anspruch 10, dadurch gekennzeichnet, dass der Ester Dimethylsulfat oder p-Toluolsulfonsäuremethylester ist.

13. Mittel zur Bekämpfung phytopathogener Pilze, gekennzeichnet durch einen Gehalt an einer Verbindung nach den Ansprüchen 1 bis 9 neben üblichen Hilfs- und/oder Trägerstoffen.

14. Verwendung einer Verbindung nach den Ansprüchen 1 bis 9 bei der Bekämpfung phytopathogener Pilze, insbesondere von Pilzen der Gattung *Pyricularia oryzae* an Reis, insbesondere Saatreis.

## Claims

1. Compounds of general formula (I):

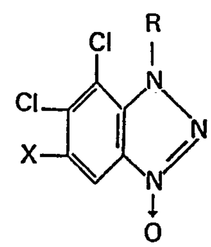

(I)

wherein R represents a straight-chained or branched alkyl group with 1 to 4 carbon atoms or allyl, and X represents a hydrogen or chlorine atom.

2. Compounds of general formula (I), wherein X represent a hydrogen atom.

3. 3-Methyl-4,5-dichlorobenzotriazole-1-N-oxide.

4. 3-Ethyl-4,5-dichlorobenzotriazole-1-N-oxide.

5. 3-n-Butyl-4,5-dichlorobenzotriazole-1-N-oxide.

6. 3-Methyl-4,5,6-trichlorobenzotriazole-1-N-oxide.

7. 3-Ethyl-4,5,6-trichlorobenzotriazole-1-N-oxide.

8. 3-Isopropyl-4,5,6-trichlorobenzotriazole-1-N-oxide.

9. 3-Allyl-4,5-dichlorobenzotriazole-1-N-oxide.

10. Process for preparing compounds as claimed in Claims 1 to 9, characterised in that

(*a*) a compound of general formula (IIa) or (IIb):

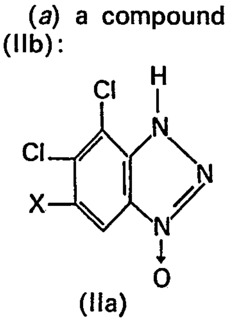

or

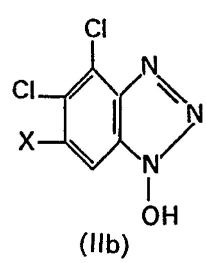

(IIa) (IIb)

wherein X is as hereinbefore defined, is heated for several hours with a sulfuric or sulfonic acid ester serving for the introduction of the group R,

appropriately in an inert solvent, and the reaction product is isolated, or

(*b*) a compound of general formula (III):

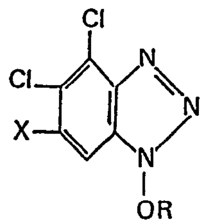

(III)

wherein R and X are as hereinbefore defined, is heated in the presence of a catalytic quantity of a sulfuric or sulfonic acid ester or a conventional phase transfer catalyst.

11. Process as claimed in Claim 10, part (*b*), characterised in that the sulfuric or sulfonic acid ester used as catalyst is derived from an alcohol of formula ROH, wherein R has the same meaning as in the starting material of general formula (III) used.

12. Process as claimed in Claim 10, characterised in that the ester is dimethyl sulfate or methyl p-toluenesulfonate.

13. Composition for combating phytopathogenic fungi, characterised in that it contains a compound as claimed in Claims 1 to 9 together with conventional excipients and/or carriers.

14. Use of a compound as claimed in Claims 1 to 9 in controlling phytopathogenic fungi, particularly fungi of the genus *Pyricularia oryzae* in rice, particularly seed rice.

## Revendications

1. Composés de formule générale (I):

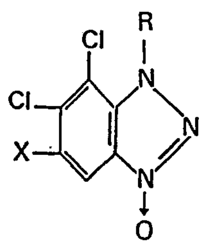

(I)

dans laquelle R représente un reste alcoyle rectiligne ou ramifié ayant de 1 à 4 atomes de carbone ou un radical allyle, et X représente un atome d'hydrogène ou de chlore.

2. Composés de formule générale (I), dans laquelle X représente un atome d'hydrogène.

3. Le 3-méthyl-4,5-dichlorobenzotriazol-1-N-oxyde.

4. Le 3-éthyl-4,5-dichlorobenzotriazol-1-N-oxyde.

5. Le 3-n-butyl-4,5-dichlorobenzotriazol-1-N-oxyde.

6. Le 3-méthyl-4,5,6-trichlorobenzotriazol-1-N-oxyde.

7. Le 3-éthyl-4,5,6-trichlorobenzotriazol-1-N-oxyde.

8. Le 3-isopropyl-4,5,6-trichlorobenzotriazol-1-N-oxyde.

9. Le 3-allyl-4,5-dichlorobenzotriazol-1-N-oxyde.

10. Procédé de préparation de composés selon les revendications 1 à 9, caractérisé par le fait que l'on chauffe

a) un composé de formule générale (IIa) ou (IIb):

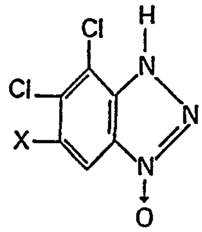

ou

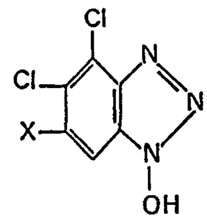

(IIa) (IIb)

formules dans lesquelles X a la signification indiquée ci-dessus, avec un ester sulfurique ou sulfonique correspondant à l'introduction du reste R, avantageusement au sein d'un solvant inerte, pendant plusieurs heures, le produit de la réaction étant isolé, ou

b) un composé de formule générale (III):

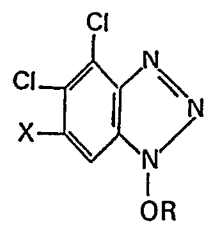

(III)

dans laquelle R et X ont la signification indiquée ci-dessus, en présence d'une quantité catalytique d'un ester sulfurique ou sulfonique ou d'un catalyseur de transfert de phases usuel.

11. Procédé selon la revendication 10, section b, caractérisé par le fait que l'ester sulfurique ou sulfonique utilisé comme catalyseur dérive d'un alcool de formule ROH, dans laquelle R a la même signification que dans la matière de départ mise en œuvre de formule générale (III).

12. Procédé selon la revendication 10, caractérisé par le fait que l'ester est constitué par du sulfate de diméthyle ou par l'ester méthylique de l'acide p-toluènesulfonique.

13. Agent pour la lutte contre les champignons phytopathogènes, caractérisé par une teneur en un composé selon les revendications 1 à 9, en plus des substances auxiliaires et/ou de support usuelles.

14. Application d'un composé selon les revendications 1 à 9 à la lutte contre les champignons phytopathogènes, notamment des champignons de l'espèce *Pyricularia oryzae* sur le riz, en particulier sur le riz d'ensemencement.